(19) Europäisches Patentamt
European Patent Office
Office européen des brevets

(11) EP 2 157 079 B1

(12) **EUROPEAN PATENT SPECIFICATION**

(45) Date of publication and mention of the grant of the patent:
**02.01.2013 Bulletin 2013/01**

(21) Application number: **09167997.7**

(22) Date of filing: **17.08.2009**

(51) Int Cl.:
***C07C 211/49*** *(2006.01)*
***C08G 18/10*** *(2006.01)*
***C08G 59/50*** *(2006.01)*
***C08L 63/00*** *(2006.01)*
***C09D 163/00*** *(2006.01)*
***C09D 175/02*** *(2006.01)*
***C09J 163/00*** *(2006.01)*

(54) **N-Alkylated 4-aminobenzyl-4-aminocyclohexanes as curing agents in polymer compositions**

N-Alkylierte 4-Aminobenzyl-4-aminocyclohexane als Härtungsmittel in Polymerzusammensetzungen

Aminocyclohexanes-4 aminobenzyl-4 N-alkylés en tant qu'agent de durcissement dans des compositions de polymère

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.08.2008 US 196535**

(43) Date of publication of application:
**24.02.2010 Bulletin 2010/08**

(73) Proprietor: **AIR PRODUCTS AND CHEMICALS, INC.**
**Allentown, PA 18195-1501 (US)**

(72) Inventors:
- **Vedage, Gamini, Ananda**
  **Bethlehem, PA 18017 (US)**
- **Thompson Thurau, Courtney**
  **Harleysville, PA 19438 (US)**
- **Conner, Mark, David**
  **New Tripoli, PA 18066 (US)**
- **O'Connell, Ellen, Margaret**
  **Orefield, PA 18069 (US)**

(74) Representative: **Westendorp | Sommer**
**Uhlandstrasse 2**
**80336 München (DE)**

(56) References cited:
**EP-A- 0 105 146**
**EP-A- 0 554 823**
**EP-A- 0 570 173**
**US-A- 2 511 028**
**US-A- 4 801 674**
**US-A- 5 312 886**
**US-A- 5 414 067**
**US-A- 5 516 872**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 157 079 B1

## Description

### BACKGROUND OF THE INVENTION

Disclosed herein are aliphatic secondary diamine curing agents that can be used, for example, in polyurea, polyurethane, urea/urethane hybrid elastomeric, epoxy resins, epoxy adhesives and composites thereof, and/or coating composition.

In particular, the present invention refers to alkylated 4-aminobenryl-4-aminocyclohexane curing agents and polymeric compositions comprising same, as well as to a method for preparing said polymeric composition. The invention also refers to polymeric compositions comprising an epoxide and a curing agent according to the invention.

The term "polymeric compositions", as used herein, describes compositions comprising 2 or more repeating units. Specific examples of polymeric compositions include, but are not limited to, polyureas, polyurethanes, urea/urethane hybrid elastomer, epoxy resins, epoxy adhesives and composites thereof, or coating compositions. Certain polymeric compositions such as polyurea elastomers are rapid cure coatings that have gel times that can be as short as 2-3 seconds. Because of its rapid cure speed, these polyurea coatings can be applied over a broad range of temperatures, are relatively moisture insensitive, and can be used on a wide variety of substrates. In addition to its application benefits, the fast cure speed may allows end-users and facility owners to return areas to service much faster than with other coatings systems, saving time and money for both the contractors and owners. These benefits, among others, have all led to significant growth in the polyurea industry over the last two decades.

There are many examples of polymeric compositions in both the patent and scientific literature as well as many commercial systems that use these coatings. Polymeric compositions such as polyurea coatings can be formed by reacting an isocyanate component with an isocyanate reactive component such as, for example a resin blend. The isocyanate component may be generally comprised of a monomer, polymer, or any variant reaction of isocyantes, quasi-prepolymer, prepolymer, or combinations thereof. The prepolymer or quasi-prepolymer can be made of an amine-terminated polymer resin, a hydroxyl-terminated polymer resin, or combinations thereof. The isocyanate reactive component or resin blend may be generally comprised of amine-terminated polymer resins, amine-terminated curing agents, hydroxyl-terminated polymer resins, hydroxyl-terminated curing agents, and combinations thereof. The term "curing agent" as used herein describes a compound or mixture of compounds that is added to a polymeric composition to promote or control the curing reaction. In certain systems, the term "curing agent" may also describe chain extenders, curatives or cross-linkers. Currently, polymeric compositions use mainly low-molecular weight diamines as curing agents such as polyoxyalkylene polyamines, cycloaliphatic diamines, or alkylates thereof. Some particular examples of these curing agents include: JEFFAMINE™ D-230 and D-400, 1,4-diaminocyclohexane, isophoronediamine, 4,4'-methylenedicyclohexylamine, methanediamine, 1,4-diaminoethyl-cyclohexane, and various alkyl-substituted derivatives of these molecules. The resin blend may also include additives or other components that may not necessarily react with the isocyanate contained therein as well as, in certain systems, catalysts.

While these polymeric compositions may vary, the isocyanate component within the composition may be generally divided into two broad classes: aromatic and aliphatic. The systems defined as aromatic may use an aromatic polyisocyanate, such as 4,4'-methylene bis isocyanato benzene (MDI), and isomers and adducts thereof. The MDI adducts referred to in both the patent and scientific literature include MDI prepolymers, quasi-prepolymers (which have a mixture of prepolymer and high free MDI monomer level and may be prepared in-situ) and mixtures of MDI prepolymers and quasiprepolymers with other MDI monomer streams. MDI adducts are sometimes prepared using an MDI monomer with a high 2,4'-MDI isomer level to reduce the reactivity and increase the pot life. For spray applied applications, the later property may be referred to as gel time and/or tack-free time. The composition may also employ one or more additional aromatic components such as, for example, the following curing agents, diethyl-toluenediamine (DETDA) or dithiomethyl-toluenediamine (ETHACURE® E300).

When the isocyanate component in the polymeric composition is aliphatic, the curing agents that are used as the isocyanate reactive component are generally also aliphatic in nature. Examples of aliphatic curing agents include, but are not limited to, dialkyl-methylene bis cyclohexylamine (which are marketed under the brandname CLEAR LINK®) or the aspartic ester products from Bayer Material Science LLC (e.g., DESMOPHEN® 1220, 1420, and 1520). The remaining ingredients within the polymeric composition, which can be added to either or both the isocyanate and resin blend components and can be aromatic or aliphatic in nature, may include any number of additional components. Examples of additional ingredients in the polymeric composition may include, for example, a polyalkylene oxide (i.e., polypropylene oxide) reacted into the polyisocyanate component to provide a quasi-prepolymer and one or more amine-terminated polypropylene oxides of functionality 2.0 or higher, such as for example, the JEFFAMINE® brand of curing agents.

Aliphatic-based polymeric compositions are typically used when the end application requires the coating to be stable when exposed to ultraviolet (UV) radiation. Although the color-stability of aliphatic polyurea coatings is highly desirable, formulators frequently complain that coatings based on commercial aliphatic curing agents, such as CLEARLINK® 1000 (provided by Dorf Ketal), offer limited formulating latitude of cure-profiles and can be somewhat stiff or brittle. This stiffness can lead to chipping, cracking and other issues-particularly at low temperatures. Furthermore, the cost of raw materials for aliphatic polyurea coatings may prohibit their use for thick film coating applications.

Aromatic-based polymeric compositions, in contrast to aliphatic-based polymeric compositions, dominate the market today, partly because their cost-in-use is competitive with other high-build coating systems. One of the shortcomings associated with aromatic-based polymeric compositions, however, is that they may exhibit poor stability when exposed to ultraviolet radiation. This may become particularly problematic in applications where the polymeric composition is a coating that is continuously subjected to UV exposure, such as in roofing or bridge coatings. The resulting UV degradation of the coating is typically manifested by at least one of the following properties: a change in color, a loss in gloss, and an adverse reduction in properties such as tensile strength, tear strength and elongation. In order to overcome these UV stability issues, formulators typically use a relatively high amount of costly UV stabilizers in order to maintain the integrity and aesthetics of the coating. Another shortcoming of aromatic-based polymeric compositions is that many formulations which incorporate dialkylated curing agents such as, for example, UNILINK® 4200 (provided by Dorf Ketal) for cure-control which may exhibit poor high temperature stability as evidenced by a low glass transition temperature ($T_g$). Coatings applied from polymeric compositions that use this type of dialkylate may become gummy or soft when exposed to heat from the sun or other sources.

Based on the issues associated with both aliphatic and aromatic curing agents described above in polymeric compositions that are polyureas, there is a need for a family of curing agents which can improve the performance and application issues associated with both aromatic and aliphatic polymeric compositions. For aromatic-based polymeric compositions, it may be desirable that a curing agent may improve the UV and high temperature stability without sacrificing physical properties such as tensile strength and tear. For aliphatic-based polymeric compositions, it may be desirable that a curing agent improve the flexibility of the formulated coating while maintaining reasonable light stability at a lower overall cost.

In embodiments wherein the polymeric composition is an epoxy resin, adhesive and/or composite, the curing agent affects at least one of the following physical properties: crosslink density, glass transition temperature (Tg), and pot life. For these polymeric compositions, long pot life may be important in ensuring proper mold filling. For example, a windmill blade can be more than 30 meters long and the resin-curing agent should infuse through the fiber reinforcement before the curing kinetics cause the viscosity to rise leading to poor fiber wet-out and weak spots. In adhesive embodiments, application of adhesive coating to relatively large parts requires a long "open time" so many adhesive beads can be applied across the part, and then when the parts are fitted together the adhesive is still tacky. If the adhesive has a short pot life and cures too quickly before the parts are adhered together, it will lower the adhesive strength of the bond.

Traditionally, curing agents that are used in polymeric compositions that are epoxy resins, epoxy adhesives, and/or composites thereof offer long pot life and high Tg are based on aromatic amine curing agents. Many of these amine curing agents have health and safety concerns as well as poor processability (e.g., MDA is a solid at room temperature). Cycloaliphatic amine curing agents are considered an alternative to aromatic amine curing agents because these agents may provide similar physical properties such as $T_g$ and crosslink density as the aromatic amine curing agents. However, the cycloaliphatic amine curing agents have much shorter pot lives than aromatic amine curing agents. An amine curing agent that would maintain or extend the long pot life of an aromatic curing agent combined with the improved processability of a cycloaliphatic curing agent, while maintaining its physical properties, would fulfill a long-sought need in the market.

Based on the issues described above with respect to polymeric compositions that are epoxy resins, epoxy adhesives, and composites thereof, there is a need for a new family of curing agents which can improve the performance, pot life, and/or processing issues associated with either adhesive or composite applications. In both applications, ideally a curing agent would retain physical properties while extending pot life. This curing agent could then be used in combination with other aromatic or cycloaliphatic amines to tailor processing, pot life and physical properties, such as, but not limited to, modulus, Tg, and other properties, to suit the end-user and final application needs.

The prior art provides several examples of amine-based curing agents. U.S. Pat. No. 4,801,674 describes the alkylation of methylene dianiline (MDA) to produce secondary aromatic diamines for use as chain extenders in reaction injection molded elastomers having the following formula:

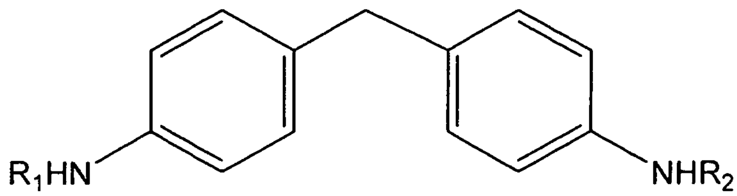

U.S. Pat. No. 5,312,886 describes secondary aliphiatic diamines of the classes: bis(4-alkylaminocyclohexyl)methane and bis(4-alkylamine-3-alkylcyclohexyl)methane having the following formula:

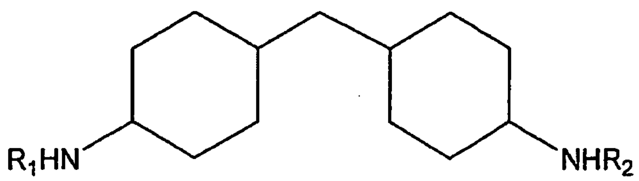

These alkylated diamines are used as chain extenders to provide light-stable polyurethane and polyurea coatings.

US 2,511,028 discloses 4-(para-aminobenzyl)cyclohexylamine.

EP 0 570 173 (A2) discloses a coating composition which is the reaction product of (I) a silane coupling agent having the formula $X_3Si(CH_2)_nY$, in which n has a value of zero to three, X is a hydrolyzable group on silicon, and Y is a reactive organofunctional group; and (ii) either a cycloaliphatic or polycycloaliphatic amine selected from aminoalkylcyclohexylamines, aminoaralkylcyclohexylamines and mixtures thereof or a cycloaliphatic or polycycloaliphatic epoxide.

EP 0554823 (A1) discloses a polyepoxide resin comprising the reaction product of a polyglycidyl polyether of a polyhydric alcohol having terminal 1,2-epoxy groups cured with a polyamine curative, the improvement which comprises the incorporation of a methylene bridged poly(cyclohexyl-aromatic)amine mixture designated MPCA represented by a particular formula.

US 5414067 (A) discloses a process for curing an epoxy resin by reacting a polyglycidyl ether of a polyhydric phenol with a cycloaliphatic polyamine curative wherein the ratio of polyglycidyl ether of a polyhydric phenol to polyamine curative is from 0.6-1.7 amine hydrogens per epoxide equivalent of polyglycidyl polyether of a polyhydric phenol.

EP 0 105 146 discloses a process for the preparation of diamines, which are optionally in the form of isomer mixtures and are optionally present together with up to 35% by weight, based on the total mixture, of the corresponding perhydrogenated diamines and/or with up to 10% by weight, based on the total mixture, of the corresponding unhydrogenated diamines, and which have a particular formula, characterized in that aromatic diamines of the formula which are optionally present in the form of isomer mixtures, are catalytically hydrogenated with the addition of 3 mol of hydrogen per mol of diamine.

US 5516872 (A) discloses a process for preparing polyurethane elastomers comprising reacting in an open mold at an isocyanate index of 90 to 110 (a) an isocyanate prepolymer having an isocyanate content of 2 to 15% by weight and having a molecular weight of from 500 to 5000, wherein said isocyanate prepolymer is a reaction product of (i) at least one organic polyisocyanate with (ii) at least one isocyanate-reactive compound having at least two isocyanate-reactive groups and having a molecular weight of from 1000 to 3000; with (b) an aminocrotonate-terminated polyether having a molecular weight of from 500 to 1500 and having a particular formula.

BRIEF SUMMARY OF THE INVENTION

Diamines which may be used as curing agents and polymeric compositions comprising these diamines which may be used, for example, in plural component coating applications and epoxy resin, epoxy adhesives, and composites thereof, are described herein. More specifically, the diamines are alkylates of 4-aminobenzyl-4-aminocyclohexane comprising a cycloaliphatic group and a cycloaromatic group. In one embodiment, there is provided a curing agent for use in a polymeric composition comprising a compound having the following Formula I:

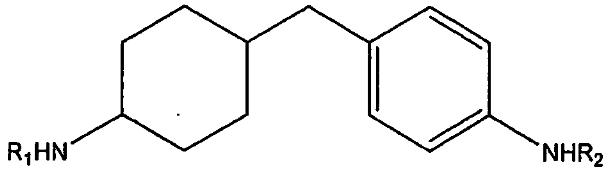

Formula I

wherein $R_1$ and $R_2$ are each a hydrogen, or an alkyl group ranging from 1 to 20 carbon atoms, wherein $R_1$ and/or $R_2$ are alkyl groups. In one embodiment, $R_1$ and $R_2$ in Formula I are the same. In another embodiment, $R_1$ and $R_2$ in Formula I are different. In yet another embodiment, $R_1$ and $R_2$ are each independently an alkyl group ranging from 1 to 12 carbon atoms.

In another embodiment, there is provided a polymeric composition comprising: an isocyanate component and a resin blend that reacts with at least a portion of the isocyanate component to provide the polymeric composition wherein the resin blend comprises a compound having the following Formula 1:

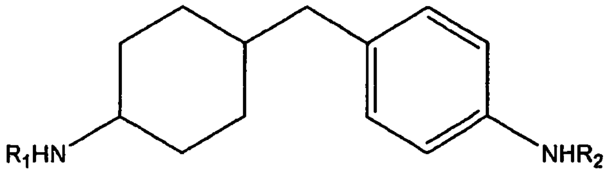

Formula I

wherein $R_1$ and $R_2$ are each a hydrogen, or an alkyl group ranging from 1 to 20 carbon atoms, wherein $R_1$ and/$R_2$ are alkyl groups.

In a further embodiment, there is provided a method for preparing a polymeric composition comprising the steps of: providing an isocyanate component; providing a resin component comprising a curing agent having a compound having the following Formula I:

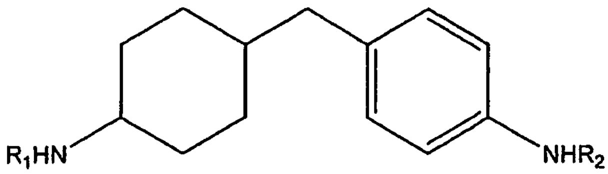

Formula I

wherein $R_1$ and $R_2$ are each a hydrogen, or an alkyl group comprising from 1 to 20 carbon atoms, wherein $R_1$ and/or $R_2$ are alkyl groups; mixing at least a portion of the isocyanate component with at least a portion of the resin component wherein at least a portion of the resin component reacts with at least a portion of the isocyanate component to provide the polymeric composition wherein the volume ratio of the isocyanate component to the resin component in the polymeric composition is any ratio in the range of from 10:1 1 to 1:10.

DETAILED DESCRIPTION OF THE INVENTION

Diamines which may be used as curing agents and polymeric compositions comprising these diamines are described herein. More specifically, the diamines described herein comprise alkylates of 4-aminobenzyl-4-aminocyclohexane comprising a cycloaliphatic group and a cycloaromatic group. The combination of the cycloaliphatic group and cycloaromatic groups may impart at least one of the following properties to the polymeric compositions made with the diamine curing agents described herein: improved UV light stability, higher tensile strength, higher modulus, longer cure profile, higher elongation, greater flexibility, and a higher $T_g$ when compared to polymeric compositions made with commercially available dialkylated curatives. For certain embodiments, the diamines described herein may enable the end-user to reduce the amount of expensive UV stabilizers used in his formulation and to improve the high temperature stability of the final coating. When used in aliphatic polymeric coating formulations, the diamine curing agents described

herein may help to decrease the $T_g$ of the coating (when compared to similar polymeric coatings formulated with alkylated bis(N-alkylaminocyclohexyl)methane curing agents), thereby improving overall coating flexibility, while maintaining reasonable color stability with a reasonable cost-in-use. While not being bound to theory, it is believed that the alkylation of 4-aminobenzyl-4-aminocyclohexane sterically hinders the diamine thereby slowing the reactivity of the curing agent enough to be applied for those embodiments wherein the polymeric composition is applied using plural component spray equipment. In addition to the advantages mentioned above, the diamine curing agents described herein may exhibit a pronounced difference in reactivities between the aromatic and aliphatic amines. This differential reactivity can be used to the coating end-user's advantage to adjust the viscosity build of a coating during cure.

As previously mentioned, the diamines described herein which may be used as curing agents in polymeric compositions to provide at least one of the following: improved elongation, high temperature resistance, UV stability and cure-profiles to polymeric compositions such as, for example, aromatic and/or aliphatic polyurea, polyurethane coatings and epoxy polymer (composites, adhesives, coatings, flooring) formulations. According to the invention, the diamine comprises an alkylate of 4-aminobenzyl-4-aminocyclohexane wherein the alkyl groups comprise from 1 to 20 carbon atoms. The alkylated diamines may exhibit a wide range of cure times depending upon the type of alkyl groups within the molecule, the degree of alkylation, and whether or not the curative is used in combination with aromatic or aliphatic isocyanates in the polymeric composition. This may provide distinct advantages in permitting the end-user to tailor the alkylated diamine to his particular cure-profile needs. Another embodiment described herein are polymeric compositions that are prepared using the alkylated diamines. It has been advantageously discovered that these aromatic polymeric compositions may provide better UV stability and high temperature stability when compared to polymeric compositions containing commercially available alkylates of methylenedianiline as curing agents. In this regard, the aliphatic polymeric compositions prepared using the alkylated diamine curing agents described herein exhibit improved flexiblity when compared to polymeric compositions prepared using bis(4-alkylaminocyclohexyl)methane curing agents, while still offering reasonable light stability at a lower cost-in-use.

In certain embodiments, the polymeric composition described herein comprises 2 or more components: an isocyanate component and an isocyanate reactive component or a resin component. In the polymeric composition, at least a portion of the resin component within the polymeric composition reacts with at least a portion of the isocyanate component. In these embodiments, the polymeric compositions, such as polyurea and polyurethane polymers, comprise an isocyanate component and a resin component, which are designated herein as an A-side and a B-side, respectively. The volume ratio of isocyanate component and resin component present within the polymeric composition may be any ratio in the range of from 10.00:1.00 to 1.00:10.00. Examples of such isocyanate and resin ratios include but are not limited to any one of the following: 10.00:1.00, 9.00:2.00, 8.00:3.00, 7.00:4.00, 6.00:5.00, 5.00:5.00, 4.00:10.00, 3.00: 9.00, 2.00:8.00, 1.00:10.00. In certain preferred embodiments, such as those applications which relate to impingement mixing, the volume ratio of isocyanate component to resin component is any ratio in the range of from 1.00:1.00 to 1.20: 1.00 isocyanate to resin. For example, the volume ratio of isocyante component to resin component may be 1.00:1.00, or 1.20:1.00, or 1.00:1.20. Examples of suitable polymeric compositions containing isocyanate and resin components are those described in U.S Pat. No. 6,403,752 . The isocyanate component may comprise a polyisocyanate which can be a monomer, a quasi prepolymer, a full prepolymer, a blend of polyisocyanates, or combinations thereof. In embodiments wherein the isocyanate component comprises a full prepolymer, a full prepolymer may be formed when the polyisocyanate is pre-reacted with a certain amount of polyamine or a polyol such that each reactive site of the polyamine or the polyol is covalently attached to one reactive site of a polyisocyanate. In these embodiments, the remaining unreacted sites of the polyisocyanate may be free to react further with the resin component or B-side within the polymeric composition. In embodiments where the isocyanate component comprises a quasi prepolymer, a certain amount of polyamine or polyol may be present in the resin or B-side that is less than that necessary to form a full prepolymer is used. The result is a mixture of prepolymer and a relatively higher amount of unreacted polyisocyanate compared to a full prepolymer. In polymeric compositions wherein the isocyanate component comprises a polyisocyanate that is monomeric or uses a quasi prepolymer, the isocyanate-reactive components in the resin component may comprise a blend of higher molecular weight components (which add flexibility to the final polymer) and lower molecular weight components (which tend to add to the strength properties of the final polymer). The term "higher molecular weight" is intended to indicate compounds having a molecular weight of greater than 400; the term "lower molecular weight" is intended to indicate compounds having a molecular weight of 400 or less. In certain embodiments, the isocyanate component may be comprised of at least 2 isocyanate groups. In these or other embodiments, it could be comprised of a dimer or trimer such as a hexamethylene diisocyanate (HDI) trimer.

Due to convenience and the application benefits of using lower viscosity components and an A to B volume ratio of about 1.00:1.00, a quasi prepolymer may be used, in certain embodiments, as the isocyanate or A component. In this or other embodiments, the polyamine or polyol that is used to form the quasi prepolymer or the full prepolymer as the isocyanate or A-side may also be used in the resin component or B-side.

Among the polyisocyanate reactants used as the polyisocyanate component (A-side), or to form the polyisocyanate component, are monomeric polyisocyanates which are at least diisocyanates. Examples of such polyisocyanates

which may be used in the polymeric compositions described herein include isophorone diisocyanate (IPDI), which is 3,3,5-trimethyl-5(isocyanato)methyl)cyclohexyl isocyanate; hydrogenated materials such as cyclohexyl diisocyanate, 4,4'-methylenedicyclohexyl diisocyanate (H12MD1); mixed aralkyl diisocyanates such as the tetramethylxylyl diisocyanates, OCN--C(CH $_3$) $_2$--C $_6$H $_4$ C(CH $_3$) $_2$-NCO; and polymethylene isocyanates such as 1 ,4-tetramethylene diisocyanate, 1,5-pentamethylene diisocyanate, 1,6-hexamethylene diisocyanate (HMDI), 1,7-heptamethylene diisocyanate, 2,2,4-and 2,4,4-trimethylhexamethylene diisocyanate, 1,10-decamethylene diisocyanate and 2-methyl-1,5-pentamethylene diisocyanate. Aromatic polyisocyanates such as phenylene diisocyanate, toluene diisocyanate (TDI), xylylene diisocyanate, 1,5-naphthalene diisocyanate, chlorophenylene 2,4-diisocyanate, bitoluene diisocyanate, dianisidine diisocyanate, tolidine diisocyanate and alkylated benzene diisocyanates; methylene-interrupted aromatic diisocyanates such as methylenediphenyl diisocyanate, the 4,4'-isomer (MDI) including alkylated analogs such as 3, 3'-dimethyl-4,4'-diphenylmethane diisocyanate and polymeric methylenediphenyl diisocyanate may also be used. It is understood that the isocyanate component is not intended to be limited to the above exemplary polyisocyanates and other isocyanates may be used.

Compositions which include oligomeric polyisocyanates (e.g., dimers, trimers, polymeric, etc.) and modified polyisocyanates (e. g., carbodiimides, uretone-imines, etc.) may also be used with the curing agents described herein in the resin side. The polyisocyanates may be used "as-is" or pre-reacted.

In one particular embodiment, the isocyanate monomer is modified by preparing a prepolymer or quasi-prepolymer of the isocyanate with an isocyanate-reactive moiety with isocyanate-reactive functionality >=2. Polyols are commonly used and can include polypropylene glycol (PPGs), polytetramethylene glycols (PTMEGs), polyethylene glycols (PEGs), polyesters, polycaprolactones and blends and copolymers of these types of isocyanate-reactive materials. As used herein, the term "polyol" refers to a single polyol or a blend of polyols. Diamines, thioethers and other isocyanate-reactive materials may also be used either alone or in combination.

The isocyanate component or A-side may also further contain various other additives which may be reactive or non-reactive to the isocyanate contained therein and/or the resin. The additional reactive components may include components such as, but not limited to, reactive diluents (e.g., propylene carbonate), plasticizers, fillers, and pigments. Non-isocyanate-reactive species are used as pigments, fillers, adhesion promoters and viscosity modifiers, for example. Other additives may include, but are not limited to, stabilizers and plasticizers.

As previously mentioned, the polymeric composition also comprises a resin or a B-side component. The resin component may be composed of components where at least a portion of the resin reacts with at least a portion of the isocyanate component contained therein. The resin component may also comprise various other additives such as, but not limited to, pigments, adhesion promoters, fillers, light stabilizers, catalyst, and combinations thereof wherein the resin component may or may not react with. The isocyanate component(s) within the polymeric blend discussed herein are reacted or cured with a resin blend comprising the curing agent disclosed herein . Curing may occur either with the diamine alone, or in combination with other polyamines or polyols such as those described below. According to the invention, the structure of curing agent described herein has the following formula I:

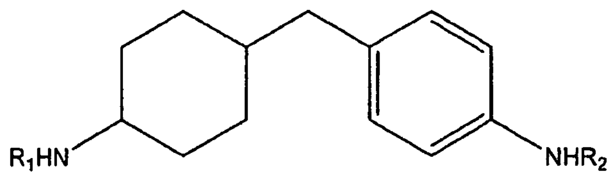

Formula I

In Formula I, groups $R_1$ and $R_2$ may each be hydrogen, or alkyl groups $R_1$ and/or $R_2$ are alkyl groups, the alkyl groups be either linear and branched alkyl groups comprising each of which from 1 to 20, or from 2 to 12, or from 2 to 6 carbon atoms. Representative alkyl groups include methyl, ethyl, propyl, isopropyl, butyl, isobutyl, secondary butyl, tertiary butyl, and the various isomeric pentyl, hexyl, heptyl, octyl, nonyl, and decyl groups. In certain embodiments, $R_1$ and $R_2$ are the same. In other embodiments, $R_1$ and $R_2$ are different. In one particular embodiment, $R_1$ and $R_2$ are each alkyl groups comprising at least three carbons. It is believed that the larger and bulkier the alkyl group used as $R_1$ and/or $R_2$ in Formula I, the slower the cure profile of the curing agent. In a further embodiment, such as when higher degrees of cross-linking and hydrogen bonding are desired, $R_2$ will be a hydrogen atom and $R_1$ will be an alkyl group.

In certain embodiments, the diamine having Formula I is added to the resin component or B-side within the polymeric composition. The isocyanate-reactive components in the resin component or B-side of the polymeric composition are typically higher molecular weight polyamines and/or polyols coupled with lower molecular weight polyamines and/or polyols that are used as curing agents and/or crosslinkers but may also further include other isocyanate-reactive components such as polythiols, polycarboxylic acids, and polyesters for example. Representative higher molecular

weight polyamines are polyoxyalkyleneamines and representative higher molecular weight polyols are polypropylene glycols. There are many different types of combinations of A-sides and B-sides possible; therefore, the final reaction product or polymeric composition may be a pure polyurea, a mixture of a polyurea and a polyurethane (a hybrid), or a polyurethane. The choice of one type over another may depend on certain factors such as application, processing parameters, and/or cost.

The isocyanate-reactive polyamines and polyols that are typically used in making polymeric compositions such as polyurethanes, polyurea-polyurethane hybrids, and polyurea polymers may range in molecular weight from about 60 to over 6,000 or from about 60 to about 5,000. Among the attributes conferred by these materials are that the higher molecular weight materials generally improve the flexibility of the final polymer and the lower molecular weight materials generally contribute to the strength properties of the final polymer. Component selection depends on many factors such as, but not limited to, handling, formulation compatibility, and end-use. The higher molecular weight polyols show a wide diversity but otherwise are rather well known and are usually dihydric, with trihydric and higher polyhydric polyols used to a lesser degree. Examples of suitable higher molecular weight polyols include poly(ethyleneoxy) glycols generally, poly(propyleneoxy) glycols, poly(butyleneoxy) glycols generally, and the polymeric glycol from caprolactone, commonly known as polycaprolactone. Other polyhydroxy materials of higher molecular weight which may be used are polymerization products of epoxides, such as ethylene oxide, propylene oxide, butylene oxide, styrene oxide, and epichlorohydrin, with materials having reactive hydrogen compounds, such as water and, more particularly, alcohols, including ethylene glycol, 1,3-and 1,2-propylene glycol, dipropylene glycol, dibutylene glycol trimethylolpropane, etc. Amino alcohols may be made, for example, by condensing amino-containing compounds with the foregoing epoxides, using such materials such as ammonia, aniline, and ethylene diamine.

Hydroxyl-containing polyesters, polythioethers, polyacetals, polycarbonates, and polyester amides also may be used instead of, or together with, the foregoing polyols. Suitable polyesters include the reaction product of polyhydric alcohols and polybasic, preferably dibasic, carboxylic acids. The polyhydric alcohols which are often used include the dihydric alcohols mentioned above. Examples of dicarboxylic acids include succinic acid, adipic acid, suberic acid, azelaic acid, sebacic acid, glutaric acid, phthalic acid, maleic acid, and fumaric acid. Hydroxyl-containing polyacetals, polycarbonates, and polyesteramides are less frequently employed in the preparation of polymeric coatings and elastomers. However, these are sufficiently well known to those practicing the art and need not be further elaborated upon here.

Lower molecular weight polyols may be added to the B-side to serve as co-curatives along with the diamines described herein. Representative examples are ethylene glycol, 1,2-propylene glycol, 1,3-propylene glycol, 1,4-and 2,3-butylene glycol, 1,6-hexanediol, 1,8-octanediol, neopentyl glycol, cyclohexane dimethanol, 2-methyl-1,3-propanediol, glycerol, trimethylolpropane, 1,2,6-hexanetriol, 1,2,4-butanetriol, pentaerythritol, mannitol, sorbitol, diethylene glycol, triethylene glycol, tetraethylene glycol, and N,N,N',N'-tetrakis(2-hydroxypropyl)ethylene diamine. Some additional examples of lower molecular weight polyols are poly(ethyleneoxy) glycols generally, poly(propyleneoxy) glycols generally, and similar poly(alkyleneoxy) glycols with molecular weights of roughly 400 or less. There are also many other types of polyols that may be used as co-curatives with the diamines disclosed herein in either lower or higher molecular weights.

The higher molecular weight polyamines used in polyurea, polyurea-polyurethane hybrid, and polyurethane formulations are well known to those skilled in the art but will be mentioned here, though not in great detail, and include diamines, triamines, and possibly higher polyfunctional amines which are primary amines. In certain embodiments, the polymeric compositions further comprise a class of polyamines having the formula $H_2N$--Y--$NH_2$. In this or other embodiments, Y is an alkylene chain and in a larger group Y is a poly(alkyleneoxy) or a polyester moiety with an alkylene group at both termini. In the foregoing, the compounds are amine-capped polyols which are the reaction product of a polyol and then an amine with alkylene oxides as well as amine-capped hydroxyl-containing polyesters. Materials of molecular weight in the 200-6000 range are most often utilized. Tri-and higher polyamines of structures similar to those in the foregoing paragraph also may be utilized.

Several common polyamines are part of a series known as JEFFAMINES™ available from Huntsman Chemical Company; examples include JEFFAMINE™ T-5000, a polypropylene oxide triamine of about 5000 molecular weight, and JEFFAMINE™ D-2000, a polypropylene oxide diamine of about 2000 molecular weight.

There are numerous ways in the art to prepare the primary precursor amine 4-aminobenzyl-4-aminocyclohexane. In one embodiment, one hydrogenates methylenedianiline (MDA) and uses a catalyst such as rhodium or ruthenium to provide the 4-aminobenzyl-4-aminocyclohexane product. Once MDA is partially hydrogenated, a distillation process such as vacuum distillation can be used to separate the 4-aminobenzyl-4-aminocyclohexane from di(4-aminocyclohexyl) methane (PACM) and MDA. In one particular embodiment, the precursor primary amine, 4-aminobenzyl-4-aminocyclohexane, is made in the following manner: A 1000 cc autoclave reactor was charged with 3.75 grams of a 4% $Rh/Al_2O_3$ catalyst and 0.28g 5% $Ru/Al_2O_3$ catalyst and 400 grams of tetrahydrofuran (THF). The reactor was purged 3 times with nitrogen and 3 times with hydrogen to remove any air from the rector and the feed. The reactor was then pressurized with hydrogen to 300 psi (2068.427 kPa) and the reactor is heated to 190°C. At this time, the pressure is adjusted to 800 pounds per square inch (psi) (5515.806 kPa) and held for 4 hours for the catalyst to get pre-reduced. At the end of the 4 hours, the reactor is cooled down, and the THF is removed from the rector and to that is added 300 grams of

methylene dianiline and 200 grams of THF. Then the reactor is heated to 180°C and 800 psi (5515.806 kPa) pressure and the hydrogenation is terminated at about 50% of the theoretical hydrogen consumptions. At this point, the product would contain the following end-products: PACM, 4-aminobenzyl-4-aminocyclohexane and MDA. Pure 4-aminobenzyl-4-aminocyclohexane was obtained by distillation of this product under vacuum.

The alkylated diamines described herein may be prepared by conventional alkylation procedures performed on the primary precursor amine or 4-aminobenzyl-4-aminocyclohexane. The alkylated diamines described herein may be prepared by any alkylation procedure performed on the precursor primary amines, a representative process of which may be found in the examples described herein. It is understood to one skilled in the art that the alkylation process can be conducting using a variety of different methods. In one embodiment, a diamine is reductively alkylated with a aldehyde or a ketone -which can be conducted in the presence or in the absence of a solvent- in the presence of a hydrogenation catalyst (such as, but not limited to, Pd, Pt, Co, Ni, Rh, or Ru) and hydrogen at elevated temperatures. In one particular embodiment, the reductive alkylation is performed by reacting a diamine and a ketone using about 2 moles of ketone with one mole of diamine, a hydrogenation catalyst as described above, and from 100 to 800 pounds per square inch (psi) (689.476 to 5515.806 kPa) hydrogen pressure at a temperature range of from 60 to 120°C.

As previously mentioned, the polymeric compositions described herein may be combined or mixed using impingement mixing directly in high-pressure application equipment. In these or other embodiments, cure time may depend not only on the type of alkyl groups on the alkylated diamines but also will depend on the amount and nature of other isocyanate-reactive materials if present in the resin component or B-side. For example, in general it will be found that the cure time as a function of $R_1$ and $R_2$ selected in the compound having Formula I increases in the order of primary alkyl<secondary alkyl<tertiary alkyl. In view of this, it should be clear that the alkylated diamines curing agents described herein can be expected to manifest an enormous range of cure times. This variability presents distinct advantages in permitting the end user to tailor the diamine to his particular needs. Since the properties of the resulting polymeric coating will also vary with the described herein, and since many diamines may be chosen with approximately the same cure time, the end user generally also will have a broad choice of diamines depending upon the performance characteristics sought for the final product.

In certain embodiments, the curing agents described herein may be used within a plural-component polyurea polymeric compositions. In these embodiments, due to the fast nature of the polyurea cure, plural-component spray equipment may frequently utilized to mix, spray and apply the A and B sides of the polymeric composition onto a substrate to provide a coating or a coated substrate. In these embodiments, the polymeric composition is produced and applied to provide a coating onto a substrate using plural component spray equipment includes two or more independent chambers for holding a isocyanate component and an resin component. Flowlines connect the chambers to a proportioner which appropriately meters the two components (A-side and B-side) to heated flowlines, which can be heated by a heater to the desired temperature and pressurized. In certain embodiments, the spray operation can be conducted at a pressure ranging from about 1,000 psi to about 3,500 psi (from about 6894.757 kPa to about 24131.651 kPa). In this or other embodiments, the spray operation can be conducted at a temperature ranging from about 120° to about 190° F (from about 48.889°C to about 87.778°C). In still further embodiments, the temperature may be as low as room temperature. Once heated and pressurized, the two or more components are then fed to a mixing chamber located in the spray-gun where they are impingement mixed before being sprayed through the nozzle and onto the substrate. Most coating systems which use plural component spray equipment for application have very quick cure times and begin to cure as a polymer layer on the substrate within seconds. Suitable equipment may include GUSMER® H-2000, GUSMER® H-3500, and GUSMER® H-20/35 type proportioning units fitted with an impingement-mix spray guy such as the Grace FUSION, GUSMER® GX-7 or the GUSMER® GX-8 (all equipment available from Graco-Gusmer of Lakewood, N.J.). Functionally similar equipment is available from a wide range of manufacturers.

Although plural-component spray equipment is described herein as a method of applying the light-stable polymeric compositions described herein, other methods may be used in preparing and forming the polymeric compositions. For example, the polymeric composition may be formed using compression molding or injection molding processes, such as reaction injection molding (RIM) processes. Furthermore, if formulated into a slow-cure system, the polymeric composition can be applied via other techniques, such as but not limited to, roll-on, low-pressure spray, dip, or trowel techniques.

In other embodiments, the diamine curing agents are used in polymeric compositions that are epoxy resin, epoxy adhesive, epoxy coating, and epoxy composites. In these embodiments, the diamine curing agent having Formula I is partially alkylated, e.g., $R_1$ is an alkyl group and $R_2$ is a hydrogen atom. For those embodiments wherein the polymeric composition comprises an epoxy, the diamine curing agent described herein may be used be itself or alternatively combined with one or more primary or secondary amine curing agents such as any of the co-curatives or curing agents described herein or known in the art. For example, in one embodiment such as those polymeric compositions that are used in filament winding composites, the diamine described herein having Formula I may be used by itself or in combination with one or more other curing agents known in the art. In one particular embodiment, the diamine curing agent described herein is used as a curing agent for a composition comprising an epoxide. Examples of suitable epoxides

include, but are not limited to, those which are based upon phenols and aliphatic polyols. Representative phenolic epoxides typically used include glycidyl polyethers of polyhydric phenols derived from a polyhydric phenol and epihalohydrin. The resulting epoxides generally will have an epoxide equivalent weight ranging from about 100 to 1,000 or from 150 to 250. Epihalohydrins used in preparing the epoxides include epichlorohydrin and epibromohydrin and polyhydric phenols include resorcinol, hydroquinone, di(4-dihydroxyphenyl)methane (commonly referred to as bisphenol F), di(4-hydroxyphenyl)propane (commonly referred to as bisphenol A) and novolacs where the phenolic groups are bridged via methylene groups. Aliphatic epoxides such as vinylcyclohexene dioxide; 3',4'-epoxy-cyclohexylmethyl-3,4-epoxy-cyclohexane carboxylate and liquid polyglycidyl ethers of polyalcohols such as 1,4-butanediol or polypropylene glycol can also be used. Other types of epoxides which can be cured with the diamine curing agents described herein are glycidyl polyesters prepared by reacting an epihalohydrin with an aromatic or aliphatic polycarboxylic acid. Epoxides utilizing glycidyl functionality from a glycidyl amine can also be used. This glycidyl functionality may be provided by reacting a polyamine with epichlorohydrin.

In embodiments wherein the polymeric composition comprises a epoxide, the epoxides can be cured in a conventional manner by effecting reaction with the diamine curing agent described herein. In one embodiment, the amount of curing agent which is reacted with the epoxide will range from 0.6 to 1.7 times the stoichiometric or equivalent amount of epoxide resin present within the composition. In one particular embodiment, the level of curing agent to epoxide is from about 0.9 to 1.1 times the stoichiometric amount, the stoichiometric amount being one equivalent weight of epoxide per equivalent weight of amine hydrogen.

Other curing agents can be used in combination with the diamine curing agents described herein in the polymeric composition and can include, but are not limited to, aromatic polyamines such as diethyltoluenediamine, and methylenedianiline; and aliphatic amines such as di(4-aminocyclohexyl)methane (PACM), isophoronediamine, 1,3-xylylenediamine, and polyalkylenepolyamines such as diethylenetriamine and triethylenetetramine and the mixed methylene bridged poly(cyclohexylaromatic)amine, 4-(4'-aminobenzyl)cyclohexylamine (ABCHA). In many cases the amine functionality for curing is provided by a mixture of an aliphatic amine such as PACM or ABCHA or both.

In certain embodiments, the polymeric composition may further comprise conventional accelerators, plasticizers, fillers, glass and carbon fibers, pigments, solvents, etc. that are used in formulating epoxy coatings, mold compositions, lacquers, etc. Selection and amount of these additives is at the option of the formulator. The adjustment of cure temperatures and curing times for polymeric compositions comprising epoxide resins is within the discretion of the formulator. In embodiments wherein the polymeric composition further comprises an accelerator, representative accelerators which may be used include, but are not limited to, boron trifluoride amine complexes and metal fluoroborate systems, e.g. copper fluoroborate; substituted phenolics, and tertiary amines, such as imidazole, 2,4,6-tri(dimethylaminomethyl)phenol, and benzyldimethylamine.

The following examples illustrate the diamines and polymeric compositions described herein. In the following examples, unless otherwise specified, area percent gas chromatography (GC) analysis was conducted using a 25 m long with a 0.17 micron film thickness HP-5 column. With the exception of Tear Strength, the test results in Tables 1 and 2 for the physical properties of the polymeric coatings were obtained using the ASTM D-412 standard at a pull rate of 2 inches/minute (8.466667 $10^{-4}$ m/s). The tear strength was obtained using the ASTM D-624 standard. The glass transition temperature for the various polymeric compositions was measured by differential scanning calorimetry (DSC) using ASTM D696. A Byk-Gardner Color-Guide was utilized to measure the CIE tristimulus values: L*, a*, b*. The total CIELAB color difference, or delta E (Δ E), is given by the following equation:

$$\Delta E^* = [(\Delta L^*)^2 + (\Delta a^*)^2 + (\Delta b^*)^2]^{0.5}$$

EXAMPLES

Example 1: Preparation of 4-aminobenzyl-4-aminocyclohexane reductive alkylate

179.6 grams (g) of 4-aminobenzyl-4-aminocyclohexane was charged to a 1 Liter Parr reactor, followed by 2.6 g of palladium over carbon (5% Pd/C) catalyst, 2.6 g platinum over carbon (5% Pt/C) catalyst, and 525 g of acetone *(Aldrich #179124)*. The molar ratio of acetone to amine was 1.2/1. The reactor was sealed and then purged several times with $N_2$ to remove residual air. It was then purged with $H_2$ and leak-checked at 120 pound-force per square inch gauge (psig) (827.370 kPa). The stir rate was set at 800 to 1000 revolutions per minute (rpm) and the temperature of the vessel was ramped to 60°C while maintaining 300 psig (2068.427 kPa) of hydrogen. These conditions were held constant until the rate of hydrogen uptake in the reaction fell below 1 psig/minute from a 1 Liter hydrogen ballast tank. The temperature was then raised to 120°C and the hydrogen pressure increased from 500 to 800 psig (from 3447.378 to 5515.805 kPa) and maintained for 0.5-1.5 hours until the reaction was complete. The product was allowed to cool before being discharged at room temperature through a 0.2 micron fiter to remove the catalyst. The product was then

rotovaped (at 20 mm Hg and temperature of 150°C which was maintained for a minimum of .5 hours) to remove excess solvent and water.

Amine titration results showed an amine equivalent weight (AEW) of 124 grams/equivalent (g/eqv) vs. the AEW for non-alkylated 4-aminobenzyl-4-aminocyclohexane of 102 g/eqv, indicating successful alkylation had occurred. The specific gravity of the product was 0.99. The area percent GC analysis showed that the resultant alkylated 4-aminobenzyl-4-aminocyclohexane was 87.4% reductive alkylate and 4.1 % reductive dialkylate.

Example 2: Preparation of 4-aminobenzyl-4-aminocyclohexane reductive alkylate

224.3 grams of 4-aminobenzyl-4-aminocyclohexane was charged to a 1 Liter Parr reactor, followed by 1.0g Pd/C catalyst, 1.0g Pt/C catalyst, 1.0g Pt/S/C (platinum sulfur over carbon catalyst), and 150.7g of acetone *(Aldrich #179124)*. The molar ratio of acetone to amine was 2.5/1. The reactor was sealed and then purged several times with $N_2$ to remove residual air. It was then purged with $H_2$ and leak-checked at 120 psig (827.370 kPa). The stir rate was set at from 800 to 1000 rpm and the temperature of the vessel was ramped to 60°C while maintaining 300 psig (2068.427 kPa) of hydrogen. These conditions were held constant until the rate of hydrogen uptake in the reaction fell below 1 psig/minute from a 1 Liter hydrogen ballast tank. The temperature was then raised to 120°C and the hydrogen pressure increased from 500 to 800 psig (from about 3447.378 to 5518.805 kPa) and maintained for 0.5 to 1.5 hours until the reaction was complete. The product was allowed to cool before being discharged at room temperature through a 0.2 micron fiter to remove the catalyst. The product was then rotovaped (20mm Hg at 150°C and maintained for a minimum of .5 hour) to remove excess solvent and water.

Amine titration results showed an amine equivalent weight of 142 g/eqv vs. the AEW for non-alkylated 4-aminobenzyl-4-aminocyclohexane of 102 g/eqv, indicating successful alkylation had occurred. The specific gravity of the product was 0.96. The area percent GC analysis showed that the resultant alkylated 4-aminobenzyl-4-aminocyclohexane was 86.9% dialkylate and 7.1 % alkylate.

Example 3: Preparation of a Polyurea Coating Containing Alkylated 4-aminobenzyl-4-aminocyclohexane (diisopropyl reductive alkylate)

A polyurea elastomeric spray coating containing the reductive alklyate of Example 2 was prepared in the following manner. First, the amine resin component (B- component) was prepared by mixing 42% Diisopropyl 4-aminobenzyl-4-aminocyclohexane with 58% JEFFAMINE® D-2000 (provided by Huntsman Corporation). A commercially available 14.5% IPDI quasi-prepolymer (Cap 100™ provided by Specialty Products, Inc.) was used as the isocyanate component (A-component). Both the A and B components were loaded into a double-barrel pneumatic joint-filler gun fitted with a Quadro® Mixer static mix head (8.7/24 x 161 millimeters (mm) provided by Sulzer ChemTech). At a pressure of 60 pounds per square inch (psig) (413.685 kPa), the two components were shot through the gun at a 1:1 volume ratio onto a piece of release liner. The sample was allowed to cure for 2 days under ambient conditions before being force-cured in a 70°C oven for 16 hours.

A 3" x 6" (7.62 cm x 15.24 cm) piece of the coating was placed in a QUV cabinet (provided by Q-Lab, Incorporated of Cleaveland, OH) for accelerated UV exposure testing. Samples were exposed to UVA light at 340nm and 0.89 W/$m^2$ intensity for 100 hours. After exposure, the panels were measured for their color change, compared to a standard non-exposed sample. The $\Delta E$ or change in color was 6.85.

Formulation information, as well as elastomer physical properties, are provided in Table 1.

Example 4: Plural Component Spray Preparation of a Polyurea Coating Containing Alkylated 4-aminobenzyl-4-aminocyclohexane (monoisopropyl reductive-alkylate)

A polyurea elastomeric spray coating containing the reductive alkylate of Example 1 was prepared in the following manner. First, the amine resin component (B-component) was prepared by mixing 36% Monoisopropyl 4-aminobenzyl-4-aminocyclohexane with 64% JEFFAMINE® D-2000 (provided by Huntsman Corp.). A commercially available 14.5% IPDI quasi-prepolymer (Cap100™ provided by Specialty Products Inc.) was used as the isocyanate component (A-component). Both A and B components were heated to approximately 160°F (71.1°C) and sprayed onto a waxed metal panel at a pressure of approximately 2500 psi (17236.893 kPa). A GUSMER® GAP-Pro plural component air-purge impingement-mix gun was used for spraying. One 18" x 18" (45.72 cm x 45.72 cm) sheet was prepared, with half of the sheet being cured overnight (~16 hours) at 70°C and the other half being allowed to cure under ambient conditions for 2 weeks before testing. The coating had an effective gel time of 50 seconds and a tack-free time of around 5 minutes. As formulated, the surface appearance was smooth.

Formulation information, as well as elastomer physical properties, are summarized in Table 2.

Example 5: Plural Component Spray Preparation of a Polyurea Coating Containing Alkylated 4-aminobenzyl-4-aminocyclohexane (mono-isopropyl reductive-alkylate)

A polyurea elastomeric spray coating containing the 4-aminobenzyl-4-aminocyclohexane reductive alkylate of Example 1 was prepared in the following manner. First, the resin component (B-component) was prepared by mixing 36% Monoisopropyl 4-aminobenzyl-4-aminocyclohexane - with 64% JEFFAMINE D-2000 (Huntsman Corp.). A commercially available 15.2% MDI quasi-prepolymer (Polyshield SS-100™ provided Specialty Products Inc.) was used as the isocyanate component (A-component). Both A and B components were heated to approximately 160°F (71.1°C)

and sprayed onto a waxed metal panel at a pressure of approximately 2500 psi (17236.893 kPa). A Grace FUSION air-purge impingement-mix gun was utilized for spraying. One 18" x 18" (45.72 cm x 45.72 cm)sheet was prepared, with half of the sheet being cured overnight (~16 hours) at 70°C and the other half being allowed to cure under ambient conditions for 2 weeks before testing. The coating had an effective gel time of 3 seconds and a tack-free time of around 5 seconds. As formulated, the surface appearance was slightly rough and exhibited an "orange peel" effect.

Formulation information, as well as elastomer physical properties, are summarized in Table 2.

Example 6: Plural Component Spray Preparation of a Light-Stable Polyurea Coating Containing Alkylated 4-aminobenzyl-4-aminocyclohexane (Diisopropyl reductive alkylate)

A polyurea elastomeric spray coating containing the 4-aminobenzyl-4-aminocyclohexane reductive dialkylate of Example 2 was prepared in the following manner. First, the amine resin blend (B-component) was prepared by mixing 49.6% Diisopropyl Half-PACM with 50.4% JEFFAMINE® D-2000 (provided by Huntsman Corp.). A commercially available 14.5% IPDI quasi-prepolymer (Cap 100™ provided by Specialty Products Inc.) was used as the isocyanate (A-component). Both A and B components were heated to approximately 160°F (71.1°C) and sprayed onto a waxed metal panel at a pressure of approximately 2500 psi (17236.893 kPa). A GUSMER® GAP-Pro air-purge impingement-mix gun was utilized for spraying. One 18" x 18" (45.72 cm x 45.72 cm) sheet was prepared, with half of the sheet being cured overnight (~16 hours) at 70°C and the other half being allowed to cure under ambient conditions for 2 weeks before testing. The coating had an effective gel time of 17 seconds and a tack-free time of 29 seconds. As formulated, the surface appearance was smooth.

Formulation information, as well as elastomer physical properties, are summarized in Table 2.

Comparative Example A: Caulk-Gun Preparation of a Polyurea Coating Containing Clearlink 1000

A polyurea elastomeric coating containing CLEARLINK® 1000 (provided by Dorf Ketal) was prepared in the following manner. First, the amine resin blend (B-component) was prepared by mixing 52% CLEARLINK® 1000 with 48% JEFFAMINE® D-2000 (provided by Huntsman Corp). A commercially available 14.5% IPDI quasi-prepolymer (Cap 100™ Specialty Products Inc.) was used as the isocyanate (A-component). Both the A and B components were loaded into a double-barrel pneumatic joint-filler gun fitted with a Quadro Mixer static mix head (8.7/24 x 161 millimeter (mm)). At a pressure of 60 psi (413.685 kPA), the two components were shot through the gun at a 1:1 by volume ratio onto a piece of release liner. The sample was allowed to cure for 2 days under ambient conditions before being force-cured in a 70°C oven for 16 hours.

A 3" x 6" (7.62 cm x 15.24 cm) piece of the coating was placed in a QUV cabinet for accelerated UV exposure testing. Samples were exposed to UVA light at 340nm and 0.89 $W/m^2$ intensity for 100 hours. After exposure, the panels were measured for their color change, compared to a standard non-exposed sample. The $\Delta E$ or change in color was 7.21.

Formulation information, as well as elastomer physical properties, are summarized in Table 2.

Comparative Example B: Caulk-Gun Preparation of a Polyurea Coating Containing Unilink 4200

A polyurea elastomeric coating containing UNILINK™ 4200 was prepared in the following manner. First, the amine resin blend (B-component) was prepared by mixing 47% UNILINK™ 4200 (provided by Dorf Ketal) with 53% JEFFAMINE® D-2000 (provided by Huntsman Corp). A commercially available 14.5% IPDI quasi-prepolymer (Cap 100™ provided by Specialty Products Inc.) was used as the isocyanate (A-component). Both the A and B components were loaded into a double-barrel pneumatic joint-filler gun fitted with a Quadro Mixer static mix head (8.7/24 x 161 mm). At a pressure of 60 psi (413.685 kPa), the two components were shot through the gun at a 1:1 by volume ratio onto a piece of release liner. The sample was allowed to cure for 2 days under ambient conditions before being force-cured in a 70°C oven for 16 hours.

A 3" x 6" (7.62 cm x 15.24 cm) piece of the coating was placed in a QUV cabinet (provided by Q-Lab, Incorporated of Cleaveland, OH) for accelerated UV exposure testing. Samples were exposed to UVA light at 340nm and 0.89 $W/m^2$ intensity for 100 hours. After exposure, the panels were measured for their color change, compared to a standard non-exposed sample. The $\Delta E$ or change in color was 33.24.

Formulation information, as well as elastomer physical properties, are summarized in Table 2.

Comparative Example C: Plural Component Spray Preparation of Light-Stable Polyurea Coating Containing CLEARLINK® 1000

A polyurea elastomeric spray coating containing a commercially available secondary diamine or CLEARLINK® 1000 (manurfactured by Dorf Detal) was prepared in the following manner. First, the amine resin blend (B-component) was prepared by mixing 52% CLEARLINK® 1000 with 48% JEFFAMINE® D-2000 (provided by Huntsman Corp.). A commercially available 14.5% IPDI quasi-prepolymer (Cap 100™ provided by Specialty Products Inc.) was used as the isocyanate (A-component). Both A and B components were heated to approximately 160°F (71.1°C) and sprayed onto a waxed metal panel at a pressure of approximately 2500 psi (17236.893 kPa). A GUSMER® GAP-Pro air-purge impingement-mix gun was used for spraying. One 18" x 18" (45.72 cm x 45.72 cm) sheet was prepared, with half of the sheet being cured overnight (~16 hours) at 70°C and the other half being allowed to cure under ambient conditions for

2 weeks before testing. The coating had an effective gel time of 22 seconds and a tack-free time of 35 seconds. The coating had a smooth surface appearance.

Formulation information, as well as elastomer physical properties, are summarized in Table 2.

Comparative Example D: Plural Component Spray Preparation of a Polyurea Coating Containing Unilink 4200 (20725-68-13)

A polyurea elastomeric spray coating containing a commercially available secondary diamine or UNILINK™ 4200 (provided by Dorf Ketal) was prepared in the following manner. First, the amine resin blend (B-component) was prepared by mixing 53.5% UNILINK™ 4200 with 46.5% JEFFAMINE® D-2000 (provided by Huntsman Corp.). A commercially available 14.5% IPDI quasi-prepolymer (Cap 100™ provided by Specialty Products Inc.) was used as the isocyanate (A-component). Both A and B components were heated to approximately 160°F (71.1°C) and sprayed onto a waxed metal panel at a pressure of approximately 2500psi (17236.893 kPa). A Grace FUSION air-purge impingement-mix gun was utilized for spraying. One 18" x 18" (45.72 cm x 45.72 cm) sheet was prepared, with half of the sheet being cured overnight (~16 hours) at 70°C and the other half being allowed to cure under ambient conditions for 2 weeks before testing. The coating had an effective gel time of 45 seconds and a tack-free time of 290 seconds. The coating had a smooth surface appearance.

Formulation information, as well as elastomer physical properties, are summarized in Table 2.

TABLE 1: Summary of Caulk-Gun Casting Physical Properties

| ***Example #*** | *Example 3* | *Comp. Ex. A* | *Comp. Ex. B* |
|---|---|---|---|
| **Component A** | | | |
| Cap 100™ (14.5% IPDI) | 100 | 100 | 100 |
| **Component B** | | | |
| Diisopropyl Alkylate of Example 2 | 42% | | |
| CLEARLINK® 1000 | | 52% | |
| UNILINK™ 4200 | | | 47% |
| JEFFAMINE® D-2000 | 58% | 48% | 53% |
| **Processing** | | | |
| A:B Volume Ratio | 1:1 | 1:1. | 1:1 |
| Isocyanate Index | 1.05 | 1.05 | 1.05 |
| **Coating Physical Properties:** | | | |
| Glass Transition Temperature, $T_g$(°C) | 41 | 53 | 26 |
| Tensile Strength at Break (psi) / kPa | 2046 / 14106.673 | 2262 / 15595.941 | 1102 / 7598.023 |
| Elongation at Break (%) | 197 | 135 | 241 |
| 100% Modulus | 996 | --- | 257 |
| **ΔE (100 hrs)** | 6.85 | 7.21 | 33.0 |

As can be seen from the properties shown in Table 1 above, when formulated into an aliphatic coating formulation, the alkylate of Example 3 exhibits a cure-profile and physical properties that falls between the performance of polymeric coatings containing bis(N-alkylaminocyclohexyl)methane (Comparative Example A) and dialkyl methylenedianiline (Comparative Example B). The molecule disclosed herein in Example 3 above shows a lower glass transition temperature than a comparable polymeric coating containing the aliphatic curing agent of Comparative Example A-thereby demonstrating how the curing agents described herein can improve the flexibility and elongation of the coating. Furthermore, after 100 hours of accelerated weatherability testing, it shows very low color-change reminiscent of the 100% cycloaliphatic curative used in Comparative Example A. Although one might anticipate that the elongation of the cycloaliphatic curing agent of Comparative Example A would greatly exceed that of the curing agent described herein due to the difference in conformational mobility of the two molecules, surprisingly the opposite is true. It is believed that this is attributable to comparatively higher levels of hard- and soft-block phase mixing seen in the morphology of polymers made with the curing agent described herein when compared to the polymer of Comparative Example A. The incorporation of the cycloaromatic ring in the structure may decrease the conformational mobility of the curative, thereby leading to less efficient packing of the hard-block regions. This phase mixing may lead to fewer hydrogen bonded segements,

thereby increasing the elongation of the polymer and decreasing the Tg.

When compared with the aromatic curing agent of Comparative Example B, the curing agent described herein exhibits improved high temperature stability and performace, as exhibited through a higher $T_g$. Additionally, it significantly improves color stability compared to the 100% cycloaromatic curative of Comparative Example B. Although one might anticipate that the high temperature stability and overall physical properties of the aromatic curing agent of Example B would surpass that of the curing agents described herein since it is an inherently stiffer molecule due to the lack of conformational mobility associated with its 100% cycloaromatic moities, surprisingly the opposite is true. It is believed that this is attributable to higher levels of hard- and soft-block phase mixing seen in Comparative Example B, which may lead to fewer hydrogen bonded hard-block segments in the polymer morphology. The incorporation of a cycloaliphatic ring in the structure of the curing agents described herein may enable higher levels of conformational mobility of the curative, thereby leading to more efficient packing of the hard-block regions, more improved hydrogen bonding, and, therefore, higher tensile strength, modulus and $T_g$.

TABLE 2: Summary of Plural Component Physical Properties

| ***Example #*** | *4* | *5* | *6* | *Comp. Ex. C* | *Comp. Ex. D* |
|---|---|---|---|---|---|
| **Component A** | | | | | |
| 14.5% IPDI | 100 | | | 100 | |
| 15.2% MDI | | 100 | 100 | | 100 |
| **Component B** | | | | | |
| Mono-Isopropyl Alkylate of Example 1 | 36% | 36% | | | |
| Di-Isopropyl Alkylate of Example 2 | | | 49.6% | | |
| CLEARLINK®1000 | | | | 52% | |
| UNILINK™ 4200 | | | | | 49.6% |
| JEFFAMINE® D-2000 | 64% | 64% | 50.4% | 48% | 50.4% |
| **Processing** | | | | | |
| A:B Volume Ratio | 1:1 | 1:1 | 1:1 | 1:1 | 1:1 |
| Isocyanate Index | 1.05 | 1.13 | 1.05 | 1.05 | 1.05 |
| Gel Time (min:sec) | 50 sec | 3 sec | 17 sec | 22 sec | 45 sec |
| Tack-FreeTime (min: sec) | 5 min | 5 sec | 29 sec | 35 sec | 290 sec |
| **Physical Properties** | | | | | |
| Tensile Strength (psi) / kPa | 1830 / 12617.406 | 2171 / 14968.518 | 1300/8963.184 | 1778 / 12258.878 | 960/6618.967 |
| Elongation (%) | 131 | 173 | 400 | 147 | 624 |
| 100% Modulus | 1472 | 1450 | 640 | 1333 | 225 |
| **Tear Strength (lbf/in) / kN/m** | 364 / 63.746 | 461 / 80.733 | 413 / 72.327 | 420 / 73.553 | 222 / 38.878 |

Comparing Example 4 with Comparative Example C, the only two Examples above which were prepared using an aliphatic isocyanate, clear differences in the performance of the curing agents described herein from the commercial benchmark emerge. First, regardless of the lower degree of alkylation of the curative of Example 4, the reactivity profile of Example 4 is much slower than that of Comparative Example C. This may be a result of the differential reactivities of the aliphatic and aromatic amines of the curing agent described herein. The differential reactivites may provide a formulator greater latitude to tailor the viscosity and cure-profile of a coating. In terms of physical properties, it appears that the tensile strength and modulus of the two systems are nearly identical.

Comparing Examples 5, 6 and Comparative Example D, the coatings prepared using aromatic isocyanates, differences in performance consistent with those described above are seen. The polymer of Example 6 shows improved tensile strength and 100% modulus compared to the polymer of Comparative Example D. This may be attributed to differences in the hard- and soft-block mixing as described above. The reactivities of the two materials, however, are quite different. The dialkyl curing agent described herein exhibits a much faster cure profile due to the difference in reactivities of the two amines, with the aliphatic amine speeding the overall cure and viscosity build of the system. Again, this difference in reactivities may offer a formulator greater formulating latitude when compared to the curing agent of Comparative Example D. Comparisons between the properties shown for Example 6 and the Comparative Examples are difficult due to the different processing conditions (iso index) used in the spray trials.

Example 7: Polymeric Compositions Comprising an Epoxy

Polymeric compositions comprising a base epoxy and curing agent were developed that were suitable for filament winding or resin infusion composite applications. The polymeric compositions comprised bisphenol A diglycidyl ether epoxy resin or EPON™ 828 (provided by Hexion Specialty Chemical of Columbus OH) and various amine curing agents including the diamine curing agent described herein and are provided in Table 3. The curing agent described herein as Example 1, which was partially alkylated, was made into a polymeric composition comprising an epoxy resin (Example 7) and compared to other polymeric compositions comprising the same epoxy resin but other cycloaliphatic amines such as dimethyl PACM (Comparative Example E) PACM (Comparative Example F) and IPDA (Comparative Example G). In addition to the foregoing, the polymeric composition described herein (Example 7) was also compared to an aromatic/cycloaliphatic amine blend of DETDA/IPDA in a 60:40 ratio (Comparative Example I) and an aromatic blend (Comparative Example H) used in the composite market. All of the Examples were mixed in a 1:1 stoichiometric ratio of epoxy resin to total curing agent.

Table 4 provides the properties of the polymeric compositions and castings obtained from these polymeric compositions. The gel times were run on a Techne gel timer (provided by Techne Inc. of Burlington, NJ) at 25°C using a 150 g total mass. Mixed viscosities were run on a Brookfield viscometer at 25°C with spindle #27 varying the rpm as needed to maintain torque and measured in centipoise (cps). The physical testing was performed on castings of the various polymeric compositions. The casts were made in a thickenss of 1/8 inches (3.175 mm) in aluminum molds, cured at 80°C for 2 hours and then 150°C for 3 hours. The testing performed was the following: ASTM #D790 for Flexural, D695 for Compression and D638 for Tensile. The glass transition temperatures (Tg) were run at 10°C/min using a TA Instruments DSC 2920 Modulated DSC.

In the comparative examples, the use of the DETDA/IPDA blend (Comparative Example I) as the curing agent within the epoxy-containing polymeric composition is intended to extend the pot life of IPDA, which as a cycloaliphatic has a short pot life (150-155 minutes for a 150 gram mass at 25°C). The Ancamine 1482 (Comparative Example H) is a eutectic blend of aromatics that is used to make MDA more liquid for easier processing. Table 4 demonstrates the advantages of the use of curing agent described herein (Example 7) within the epoxy-containing polymeric composition over other curing agents within the same polymeric composition.

As can be seen in Table 4 below, the curing agent used in Example 7 provides a significant advantage in processing via a gel time of 35 hours while maintaining both physical properties and manageable viscosity for handling purposes. Depending upon the end-use, physical properties such as modulus and Tg can be further increased with the use of multifunctional epoxy resins which may increase the crosslink density.

Table 4 demonstrates the advantages of the using the curing agent described herein: much longer pot life when compared to similar polymeric compositions containing either cycloaliphatics or aromatic curing agents. In addition, when comparing Example 7 to an aromatic MDA eutectic such as ANCAMINE® 1482 (Comparative Example H), which offers the longest pot life of the comparative examples as measured by gel time, the viscosity of Example 7 is much lower, potentially allowing no or minimal heating of the components for infusion or winding processing.

TABLE 3: Polymeric Composition Formulations Comprising an Epoxy Resin

| | Ex. 7 | Comp. Ex. E | Comp. Ex. F | Comp. Ex. G | Comp. Ex. H | Comp. Ex. I |
|---|---|---|---|---|---|---|
| Epoxy Component: | | | | | | |
| EPON™828 (1) | 100 g | 100 g | 100 g | 100 g | 100 g | 100 g |
| Curing Agent Component: | | | | | | |
| Mono-Isopropyl Alkylate of Ex. 1 | 65 g | | | | | |
| DMPACM (2) | | 32 g | | | | |
| PACM (3) | | | 28 g | | | |

(continued)

| | Ex. 7 | Comp. Ex. E | Comp. Ex. F | Comp. Ex. G | Comp. Ex. H | Comp. Ex. I |
|---|---|---|---|---|---|---|
| Epoxy Component: | | | | | | |
| IPDA (4) | | | | 22 g | | |
| ANCAMINE® 1482 (5) | | | | | 25 g | |
| DETDA (6) | | | | | | 13.56 g |
| IPDA (7) | | | | | | 9.04 g |
| Stoichiometric Ratio Epoxy: Curing Agent | 1:1 | 1:1 | 1:1 | 1:1 | 1:1 | 1:1 |
| (1) EPON™ 828 (provided by Hexion Specialty Chemical of Columbus OH);<br>(2) DMPACM is dimethyl PACM provided by ANCAMINE® 2049 provided by Air Products and Chemicals, Inc. of Allentown, PA;<br>(3) PACM is AMICURE® PACM provided by Air Products and Chemicals, Inc. of Allentown, PA;<br>(4) IPDA is VESTAMIN® IPD provided by Evonik Industries (formerly Degussa) of Germany;<br>(5) ANCAMINE® provided by Air Products and Chemicals, Inc. of Allentown, PA;<br>(6) DETDA is ETHACURE® 100 provided by Albemarle Corp. of Baton Rouge, LA;<br>(7) IPDA is VESTAMIN® IPD | | | | | | |

TABLE 4: Epoxy Resin Compositions containing Different Curing Agents

| Property | Ex. 7 | Comp. Ex. E | Comp. Ex. F | Comp. Ex. G | Comp. Ex. H | Comp. Ex. I |
|---|---|---|---|---|---|---|
| Mixed viscosity (resin & curing agent), cps | 5700 | 2300 | 2700 | 1900 | 12,000 | 3300 |
| Gel time (150 gr mass, RT), min | 2100 | 330 | 160 | 150 | 590 | 250 |
| Tensile strength (MPa) | 78 | 77 | 71 | 77 | 87 | 62 |
| Tensile modulus (MPa) | 2700 | 2600 | 2400 | 2600 | 2600 | 2600 |
| Ultimate elongation (%) | 6.4 | 5.2 | 5.4 | 4.8 | 7.5 | 3.2 |
| Ultimate flexural strength, MPa | 140 | 130 | 120 | 130 | 120 | 140 |
| Flexural Modulus, MPa | 2700 | 2500 | 2200 | 2600 | N/A | N/A |
| Tg (°C) by DSC | 120 | 160 | 150 | 160 | 150 | 150 |
| *Data reported to two significant digits | | | | | | |

## Claims

1. A curing agent for use in a polymeric composition comprising a compound having the following Formula I:

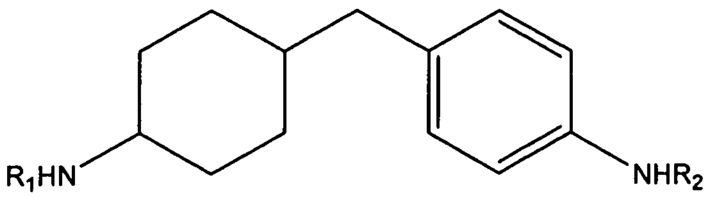

Formula I

wherein $R_1$ and $R_2$ are each a hydrogen, or an alkyl group comprising from 1 to 20 carbon atoms, wherein $R_1$ and/or $R_2$ are alkyl groups.

**2.** The curing agent of claim 1 wherein $R_1$ and $R_2$ are each independently alkyl groups comprising from 1 to 12 carbon atoms.

**3.** The curing agent of claims 1 or 2 wherein $R_1$ and $R_2$ are the same.

**4.** The curing agent of claim 1 wherein $R_1$ and $R_2$ are different.

**5.** A polymeric composition comprising:

an isocyanate component, and
a resin blend that reacts with at least a portion of the isocyanate component to provide the polymeric composition wherein the resin blend comprises a curing agent according to claim 1.

**6.** The polymeric composition of claim 5 wherein a volume ratio of isocyanate component to resin component is any ratio within the range of from 10.00:1.00 to 1.00:10.00.

**7.** The polymeric composition of claims 5 to 6 wherein the isocyanate component comprises at least one selected from the group consisting of a monomer, a quasi prepolymer, a full prepolymer, a blend of polyisocyanates, and combinations thereof.

**8.** The polymeric composition of claim 7 wherein the quasi prepolymer comprises at least one selected from the group consisting of: an aliphatic isocyanate, an aromatic isocyanate, and an active hydrogen-containing material.

**9.** The polymeric composition of claim 8 wherein the active hydrogen-containing material comprises at least one chosen from a polyol, a high molecular weight amine-terminated polyoxyalkylene polyol, and a mixture thereof.

**10.** A method for preparing a polymeric composition, the method comprising:

providing an isocyanate component;
providing a resin component comprising a curing agent according to claim 1 or 2;
mixing the at least a portion of the isocyanate component with at least a portion of the resin component wherein the at least a portion of the resin component reacts with the at least a portion of the isocyanate component to provide the polymeric composition wherein the volume ratio of the isocyanate component to the resin component in the polymeric composition is any ratio in the range of from 1.00:1.00 to 1.20:1.00.

**11.** The method of claim 10 wherein the ratio of isocyanate component to resin component is 1.00:1.00.

**12.** A polymeric composition comprising:

an epoxide, and
a curing agent according to claim 1.

**Patentansprüche**

**1.** Härtungsmittel zur Verwendung in einer Polymerzusammensetzung, umfassend eine Verbindung mit der folgenden Formel I:

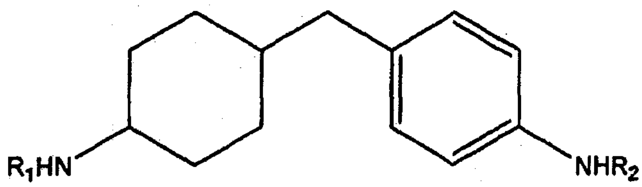

Formel I

wobei $R_1$ und $R_2$ jeweils ein Wasserstoff oder ein Alkylrest, umfassend 1 bis 20 Kohlenstoffatome, sind, wobei $R_1$ und/oder $R_2$ Alkylreste sind.

**2.** Härtungsmittel nach Anspruch 1, wobei $R_1$ und $R_2$ jeweils unabhängig voneinander Alkylreste, umfassend 1 bis 12 Kohlenstoffatome, sind.

**3.** Härtungsmittel nach den Ansprüchen 1 oder 2, wobei $R_1$ und $R_2$ gleich sind.

**4.** Härtungsmittel nach Anspruch 1, wobei $R_1$ und $R_2$ unterschiedlich sind.

**5.** Polymerzusammensetzung, umfassend:

eine Isocyanat-Komponente und
eine Harzmischung, welche sich mit mindestens einem Teil der Isocyanat-Komponente umsetzt, wobei die Polymerzusammensetzung bereitgestellt wird, wobei die Harzmischung ein Härtungsmittel gemäß Anspruch 1 umfasst.

**6.** Polymerzusammensetzung nach Anspruch 5, wobei ein Volumenverhältnis von Isocyanat-Komponente zu Harz-Komponente jedwedes Verhältnis im Bereich von 10,00:1,00 bis 1,00:10,00 ist.

**7.** Polymerzusammensetzung nach den Ansprüchen 5 bis 6, wobei die Isocyanat-Komponente mindestens eines, ausgewählt aus der Gruppe, bestehend aus einem Monomer, einem Quasi-Vorpolymer, einem Voll-Vorpolymer, einer Mischung von Polyisocyanaten und Kombinationen davon, umfasst.

**8.** Polymerzusammensetzung nach Anspruch 7, wobei das Quasi-Vorpolymer mindestens eines, ausgewählt aus der Gruppe, bestehend aus: einem aliphatischen Isocyanat, einem aromatischen Isocyanat und einem aktiven Wasserstoff-enthaltenden Material, umfasst.

**9.** Polymerzusammensetzung nach Anspruch 8, wobei das aktiven Wasserstoffenthaltende Material mindestens eines, ausgewählt aus einem Polyol, einem Aminterminierten Polyoxyalkylenpolyol mit hohem Molekulargewicht und einem Gemisch davon, umfasst.

**10.** Verfahren zur Herstellung einer Polymerzusammensetzung, wobei das Verfahren umfasst:

Bereitstellen einer Isocyanat-Komponente;
Bereitstellen einer Harz-Komponente, welche ein Härtungsmittel gemäß Anspruch 1 oder 2 umfasst;
Mischen des mindestens einen Teils der Isocyanat-Komponente mit mindestens einem Teil der Harz-Komponente, wobei sich der mindestens eine Teil der Harz-Komponente mit dem mindestens einen Teil der Isocyanat-Komponente umsetzt, wobei die Polymerzusammensetzung bereitgestellt wird, wobei das Volumenverhältnis der Isocyanat-Komponente zu der Harz-Komponente in der Polymerzusammensetzung jedwedes Verhältnis im Bereich von 1,00:1,00 bis 1,20:1,00 ist.

**11.** Verfahren nach Anspruch 10, wobei das Verhältnis von Isocyanat-Komponente zu Harz-Komponente 1,00:1,00 ist.

**12.** Polymerzusammensetzung, umfassend:

ein Epoxid und
ein Härtungsmittel gemäß Anspruch 1.

## Revendications

1. Un agent de durcissement destiné à être utilisé dans une composition de polymère comprenant un composé possédant la formule I ci-après:

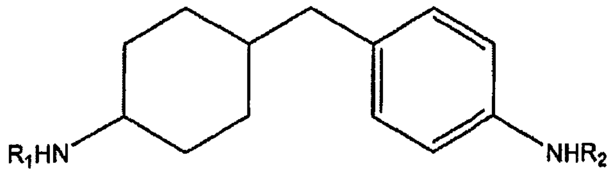

Formule I

dans laquelle $R_1$ et $R_2$ sont chacun un hydrogène, ou un groupement alkyle comportant de 1 à 20 atomes de carbone, dans laquelle $R_1$ et/ou $R_2$ sont des groupements alkyle.

2. L'agent de durcissement de la revendication 1, dans lequel $R_1$ et $R_2$ sont chacun indépendamment des groupements alkyle comportant de 1 à 12 atomes de carbone.

3. L'agent de durcissement de la revendication 1 ou 2, dans lequel $R_1$ et $R_2$ sont identiques.

4. L'agent de durcissement de la revendication 1, dans lequel $R_1$ et $R_2$ sont différents.

5. Une composition de polymère comprenant:

   un composant isocyanate et
   un mélange de résine, qui réagit avec au moins une portion du composant isocyanate pour donner la composition de polymère dans laquelle le mélange de résine comprend un agent de durcissement selon la revendication 1.

6. La composition de polymère de la revendication 5, dans laquelle un rapport en volume entre le composant cyanate et le composant résine est n'importe quel rapport situé dans l'intervalle de 10,00:1,00 à 1,00:10,00.

7. La composition de polymère des revendications 5 à 6, dans laquelle le composant isocyanate comprend au moins un élément sélectionné parmi le groupe se composant d'un monomère, d'un quasi-prépolymère, d'un prépolymère complet, d'un mélange de polyisocyanates et de combinaisons de ceux-ci.

8. La composition de polymère de la revendication 7, dans laquelle le quasi-prépolymère comprend au moins un élément sélectionné parmi le groupe se composant d'un isocyanate aliphatique, d'un isocyanate aromatique et d'une matière contenant de l'hydrogène actif.

9. La composition de polymère de la revendication 8, dans laquelle la matière contenant de l'hydrogène actif comprend au moins une sélectionnée parmi un polyol, un polyoxyalkylène polyol à terminaison amine de poids moléculaire élevé et un mélange de ceux-ci.

10. Une méthode de préparation d'une composition de polymère, la méthode comprenant:

    la mise à disposition d'un composant isocyanate;
    la mise à disposition d'un composant résine comprenant un agent de durcissement selon la revendication 1 ou 2;
    le mélangeage de la au moins une portion du composant isocyanate avec au moins une portion du composant résine, la au moins une portion du composant résine réagissant avec la au moins une portion du composant isocyanate pour donner la composition de polymère dans laquelle le rapport en volume entre le composant isocyanate et le composant résine (dans la composition de polymère) est n'importe quel rapport situé dans

l'intervalle de 1,00:1,00 à 1,20:1,00.

11. La méthode de la revendication 10, dans laquelle le rapport entre le composant isocyanate et le composant résine est de 1,00:1,00.

12. Une composition de polymère comprenant:

un époxyde et
un agent de durcissement selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4801674 A
- US 5312886 A
- US 2511028 A
- EP 0570173 A2
- EP 0554823 A1
- US 5414067 A
- EP 0105146 A
- US 5516872 A
- US 6403752 B